# EUROPEAN PATENT APPLICATION

(11) **EP 0 836 864 A2**
(43) Date of publication of application: **22.04.1998**
(21) Application number: 97308323.1
(22) Date of filing: 20.10.1997
(51) Int. Cl.: A61N 1/04

(54) **Defibrillator electrode**

(30) Priority: 18.10.1996 US 28683 P; 17.10.1997 US
(71) Applicant: Graphic Controls Corporation, Buffalo, New York 14240 (US)
(72) Inventor: Semler, John R., Clarence, New York 14221 (US); Gusakov, Ignatz, Aurora, New York 14052 (US); Solender, Peter E., Williamsville, New York 14221 (US)
(74) Representative: Pacitti, Pierpaolo A.M.E.

(57) **Abstract**

A defibrillator electrode comprising a substrate having a periphery, a foil plate adhered to the substrate, a retaining ring defining a volume adhered around the periphery of the substrate, a conductive hydrogel adhesive containing an electrolyte disposed over the foil plate within the volume of the retaining ring, and a release liner disposed over the conductive hydrogel adhesive. The electrolyte is preferrably potassium bromide. Also included is a post for making an electrical connection with the electrode. The post has a substantially cylindrical portion with an end, an indentation on the spherical portion, and a spherical ball on the end, wherein the indentation and the spherical ball are adapted to mate with corresponding portions of a plug to which the post is connected. In another aspect, the invention provides a post having a substantially disk-shaped body portion and a top portion disposed on the body portion, the body portion having a hole extending diametrically therethrough to receive a wire for establishing electrical connection with the electrode. In still another aspect, the invention provides a post having a substantially cup-shaped body portion having a wall, a groove in the wall, and a snap ring disposed in the groove. In all of these aspects, the post has a portion adapted to extend through a hole in the electrode and to be crimped thereon. The invention also includes a washer for making electrical contact between the foil plate and the post.

## Description

### FIELD OF THE INVENTION

The present invention relates to defibrillators and, in particular, to a multi-part defibrillator electrode.

### BACKGROUND OF THE INVENTION

In the treatment of medical patients, and particularly cardiac patients, it is often necessary to transfer electrical energy to and from the body of the patient for various reasons. This transfer of electrical energy is achieved through electrodes contacting the skin of the patient. These electrodes are generally classified as physiological electrodes.

There are three broad categories of physiological electrodes: monitoring devices, therapeutic devices, and stimulating devices. Examples of monitoring devices include cardioscopes and electric cardiographs for monitoring operation of the heart and impedance pnuemographs for monitoring respiration. Therapeutic devices include electrode surgical units and various radio frequency and other relatively high frequency applicators to reduce pain and promote healing. Stimulating devices include defibrillators (used to shock a patient from fibrillation, which is an asynchronous cardiac ventricular or fluttering) and other directed current and low frequency applicators. The present invention is directed to an improved defibrillator electrode.

When administering defibrillation pulses to a patient, speed and precision are frequently required because an emergency situation is typically at hand. It is therefore desirable to have a defibrillator device that is easily assembled and applied to the patient. The electrode itself should be capable of delivering as much energy as possible to the patient without dissipating that energy throughout the electrode components. The electrode should also have a rapid recovery rate to allow rapid ascertainment of results of application of a defibrillator pulse and to allow rapid application of further pulses if necessary. The present invention provides a defibrillator electrode that meets these criteria and provides further advantages over the prior art as discussed in the following description.

### SUMMARY OF THE INVENTION

The present invention provides a defibrillator electrode comprising a substrate having a periphery, a foil plate adhered to the substrate, a retaining ring defining a volume adhered around the periphery of the substrate, a conductive hydrogel adhesive containing an electrolyte disposed over the foil plate within the volume of the retaining ring, and a release liner disposed over the conductive hydrogel adhesive. The electrolyte is preferrably potassium bromide.

The invention also includes a post for making an electrical connection with the electrode. The post has a substantially cylindrical portion with an end, an indentation on the cylindrical portion, and a spherical ball on the end, wherein the indentation and the spherical ball are adapted to mate with corresponding portions of a plug to which the post is connected.

In another aspect, the invention provides a post having a substantially disk-shaped body portion and a top portion disposed on the body portion, the body portion having a hole extending diametrically therethrough to receive a wire for establishing electrical connection with the electrode.

In still another aspect, the invention provides a post having a substantially cup-shaped body portion having a wall, a groove in the wall, and a snap ring disposed in the groove. In all of these aspects, the post has a portion adapted to extend through a hole in the electrode and to be crimped thereon. The invention also includes a washer for making electrical contact between the foil plate and the post.

It is to be understood that both the foregoing general description and the following detailed description are exemplary, but are not restrictive, of the invention.

### BRIEF DESCRIPTION OF THE DRAWINGS

Fig. 1 is a perspective assembly view of an exemplary electrode according to the present invention;
Fig. 2 is a top view of an exemplary component of an electrode according to the present invention;
Fig. 3 is a schematic of an exemplary connector structure for use with the electrode of the present invention;
Fig. 4A is a top view of an alternative embodiment of the electrode component shown in Fig. 2;
Fig. 4B is a sectional view of an alternative embodiment of the exemplary electrode component shown in Fig. 2;
Fig. 4C is a top view of another alternative embodiment of the electrode component shown in Fig. 2;
Fig. 5 is top view of an alternative embodiment of the electrode according to the present invention;
Fig. 5A is a top view of an exemplary component used in the electrode shown in Fig. 5;
Fig. 6 is a side assembly view of the electrode shown in Fig. 5;
Fig. 7 is a side sectional view taken along line 7-7 of the electrode shown in Fig. 5;
Fig. 8 is a top view of another alternative embodiment of the electrode according to the present invention;
Fig. 9 is a side assembly view of the electrode shown in Fig. 8;
Fig. 10 is a side sectional view taken along line 10-10 of the electrode shown in Fig. 8;
Fig. 11 is a top view of a component of the exemplary electrode of Fig. 8;
Fig. 12 is a side view of the component shown in Fig. 11;
Fig. 13 is a side assembly view of another alternative embodiment of the electrode according to the present invention;
Fig. 14 is a side sectional view of the electrode shown in Fig. 13;
Fig. 15 is a side sectional view of a component of the electrode shown in Fig. 14; and
Fig. 16 is a schematic illustration of an exemplary experimental circuit used to test the electrodes of the present invention.

### DETAILED DESCRIPTION OF THE INVENTION

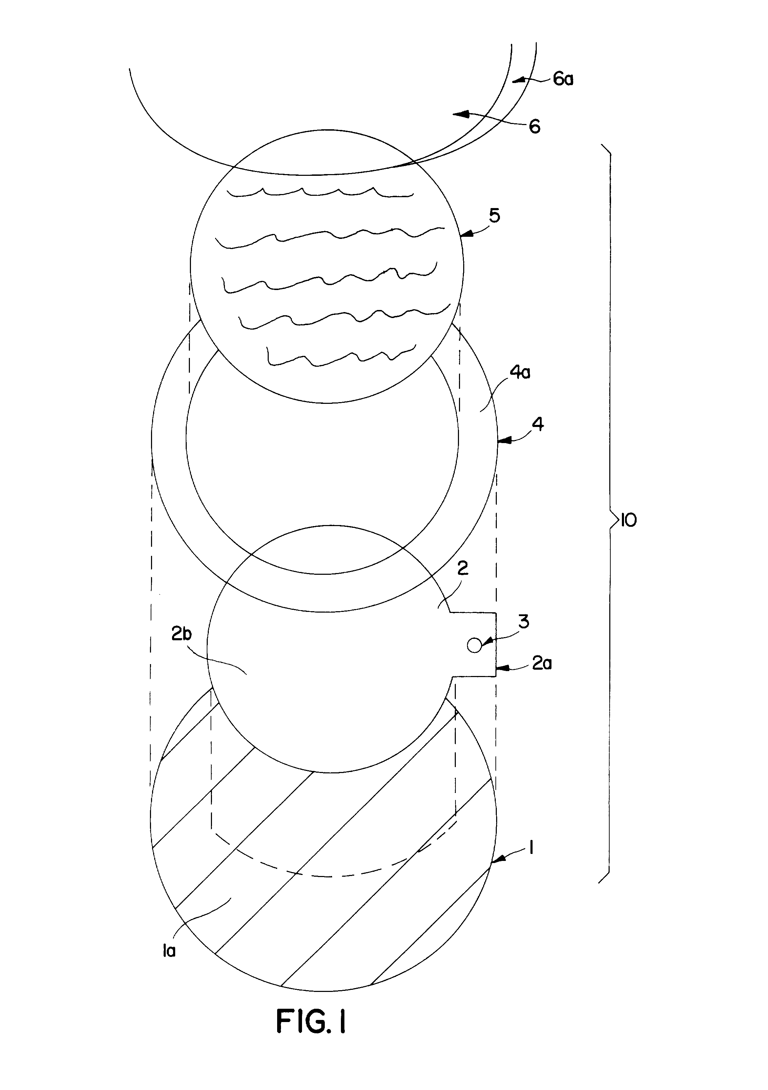

Fig. 1 shows a perspective assembly view of an exemplary electrode 10 according to the present invention. The exemplary embodiment shown in Fig. 1 includes a foam or vinyl insulating backing or substrate 1. Substrate 1 in the illustrated embodiment is in the shape of a circle and has a conventional adhesive on one side (patient side la) only. Substrate 1 has the following dimensions in the exemplary embodiment shown (although it is well within the skill in the art to develop substrates and other components discussed herein, with other shapes and dimensions): a diameter of 6.125 inches (15.5 cm) and a thickness of 40 mils (0.1 cm). Although both materials are suitable for substrate 1, vinyl is preferable to foam because it costs less (about 1/3 the cost of foam).

A foil plate 2 (made of tin metal in the exemplary embodiment shown), with an extending tab 2a (also made of tin metal in the exemplary embodiment shown), is secured to patient side la of, and is centered on, substrate 1. Foil plate 2 has a diameter of 3.625 inches (9.2 cm) and a thickness of 2 mils (0.005 cm) in the exemplary embodiment shown, and tab 2a has a length of 1.5 inches (3.8 cm) and a width of 1.375 inches (3.5 cm). (All dimensions herein are exemplary only.) Alternatively, tab 2a may be omitted. The outer dimension of foil plate 2 may be increased to include the area previously occupied by the metal tab 2a in such an alternative embodiment. Foil plate 2 is secured to substrate 1 by the adhesive on patient side la of substrate 1.

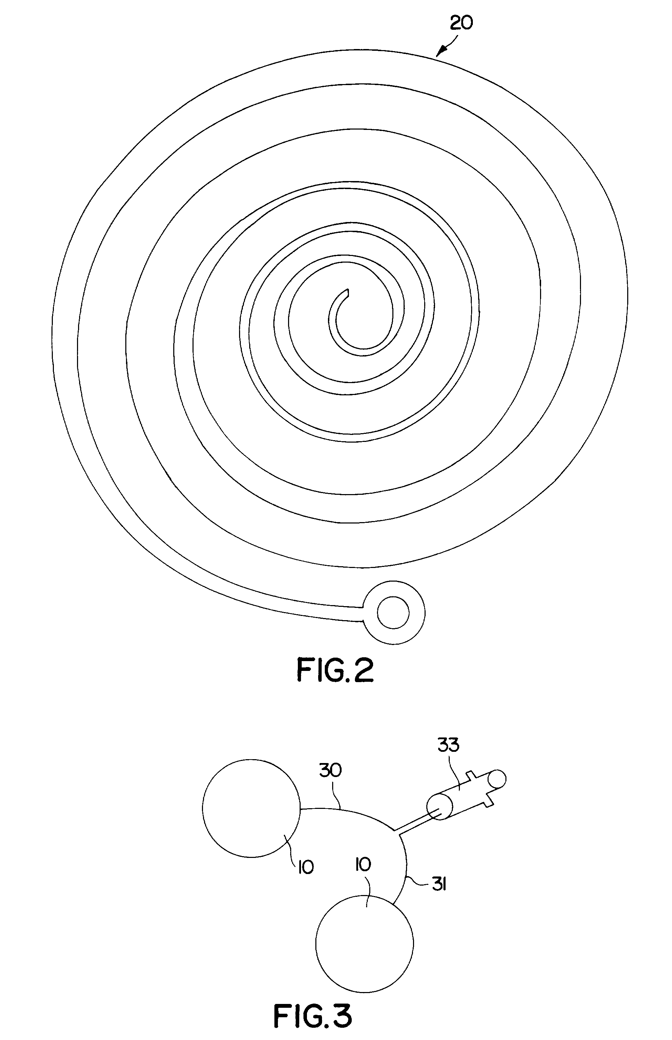

As an alternative to foil plate 2, a spiral 20 can be used as shown in Fig. 2. A wire or ribbon is formed into spiral 20 by coiling, by printing with a conductive ink, by etching using a photoetch process, or by a combination of these methods, all of which are known techniques in the art.

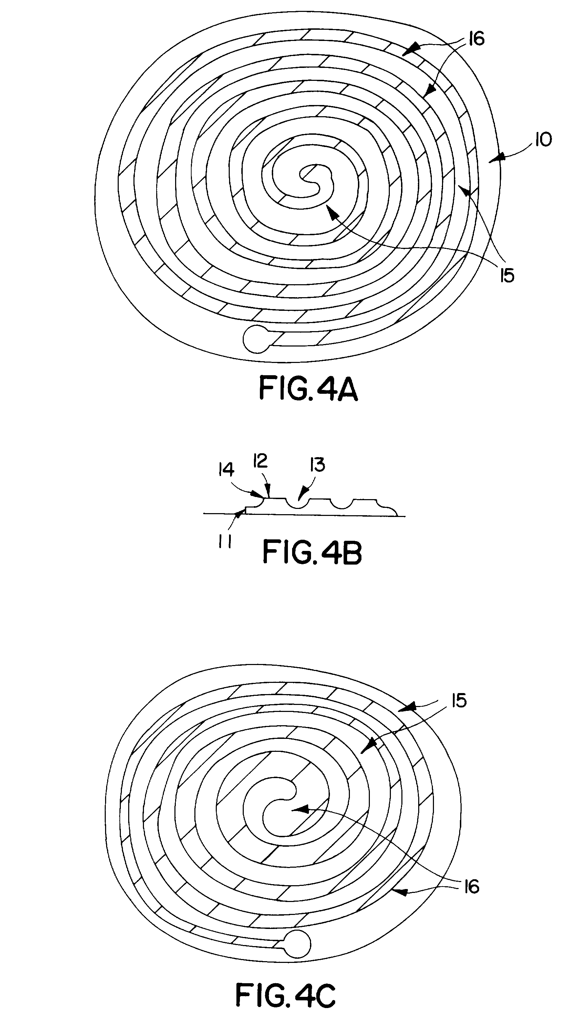

Fig. 4B illustrates an embodiment wherein a thermoformed, thin-film, flexible substrate 11 including peaks 12 and valleys 13 formed using a photoetch process (wherein peaks 12 form the spiral 20 as shown in Fig. 2). A conductive ink 14 is printed on peaks 12.

As shown in Fig. 4A, coil 20 may be of constant width, with the spacing 15 between each of the coil loops 16 being variable (in Fig. 4A, spacing 15 decreases from the center outward). Alternatively, the width of each coil loop 16 may vary, with a constant width spacing 15 between each loop, as shown in Fig. 4C. Both designs (as illustrated in Figs. 4A and 4C) are intended to control the current density throughout the electrode surface, which reduces the potential for high current densities to build in concentrated regions of the electrode. High current densities may burn the patient, damage heart muscle, or both. Different patterns may perform the same function, as will be understood by those skilled in the art.

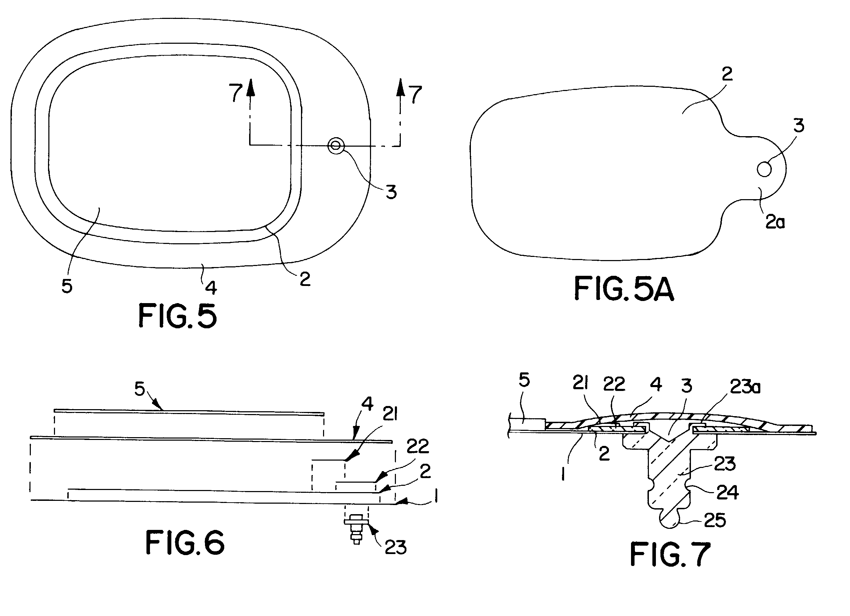

Referring back to Fig. 1, a snap connector (not shown) is connected to foil plate 2 through hole 3 in the center of tab 2a (or near the edge of foil plate 2 if tab 2a is not included). The snap connector makes the electrical connection between an electrical source and electrode 10. The snap connector may be a female connector identical to that employed in a Laerdal Heartstart defibrillation electrode product, which is compatible with Laerdal cables. The dimensions of such a snap connector in an exemplary embodiment are as follows: an external diameter of 0.5 inches (1.27 cm), an internal diameter of 0.3125 inches (0.8 cm), and a depth of 0.25 inches (0.64 cm). Foil plate 2 is fastened to the snap connector via a rivet (not shown) sandwiching foil plate 2 between the base of the snap connector, a clear plastic disk (not shown), and a metal washer (not shown). Alternatively, a male snap connector post compatible with the defibrillator cables disclosed in U.S. Patent No. 4,671,591 is suitable for use as the snap connector. Alternative snap connector means are discussed in detail below in connection with Fig. 5.

A foam retaining ring 4 of the same diameter as substrate 1 is centered on the periphery of substrate 1 and secured to patient side la of substrate 1 by the adhesive on patient side la. Foam retaining ring 4 has an external diameter of 6.125 inches (15.5 cm), an internal diameter of 4.125 inches (10.5 cm), a width of 1.0 inch (2.5 cm), and a thickness of 40 mils (0.1 cm) in the exemplary embodiment shown. Foam retaining ring 4 also has adhesive on patient side 4a. Retaining ring 4 provides the advantages of forming a mold to hold hydrogel 5. This makes construction of the electrode easier because there is a confined area in which to insert hydrogel 5. Retaining ring 4 forms a wall so hydrogel 5 can be poured inside it.

Conductive hydrogel 5 adheres to patient side 2b of foil plate 2 and is centered on foil plate 2. Hydrogel 5 has a diameter of 4.125 inches (10.5 cm) and weighs 25 grams in the exemplary embodiment shown. Three alternative UV-cured hydrogels are preferred for use as hydrogel 5. Theses three alternatives are a urethane oligomer system, a gel available from Graphic Controls' of Buffalo, N.Y. using the designation Gananoque M1-112 gel, and an acrylic acid/carboxyethylacrylate copolymer system such as that described in U.S. Patent No. 5,614,586, which is incorporated herein by reference for its teaching regarding conductive hydrogel adhesives. The acrylic acid/carboxyethylacrylate system is most preferred, and has the following exemplary formulation:

| **Component** | **Percentage (Function)** |
|---|---|
| Water | 45.65 (Solvent) |
| KBr | 4.00 (Electrolyte) |
| KOH | 6.35 (Neutralizing Agent-pH) |
| Acrylic Acid | 7.00 (Monomer) |
| Hydroxyethyl Cellulose | 0.80 (Thickening Agent) |
| Glycerin | 20.00 (Humectant) |
| B. Carboxyethyl Acrylate | 14.00 (Oligomer) |
| 3% Methylene Bisacrylamide in Propylene Glycol | 2.00 (Cross-Linking Agent) |
| Darocur 1173 | 0.20 (photoinitiator) |

Darocur 1173 is 2-hydroxy-2-methyl-1-phenyl-propane-1-one (available from EM Industries Inc. of Hawthorne, New York 10532) and serves as the photoinitiator in the UV polymerization reaction. The viscosity of this exemplary system is about 2585 cps and the pH is about 5.06. Potassium bromide is used as the electrolyte in the gel to enhance conductivity. In an alternative embodiment, potassium chloride may be substituted for potassium bromide.

By virtue of the components used therein, hydrogel 5 provides less dry out and better rate of recovery after defibrillation than known gels. Recovery is the ability of the system to respond from the high energy defibrillation charge, which throws off the equilibrium and tends to cause plating out of the potassium and bromide (or other electrolyte component) at the electrodes, which is undesirable. Use of the components of this invention prevents such plating out, allowing the electrolyte to stay on the surface of hydrogel 5. Hydrogel 5 also provides lower resistance than known hydrogels, which allows application of as much energy as possible to the patient without losing the energy through the electrode.

A polyethylene release liner 6 is placed over hydrogel 5 in the embodiment illustrated in Fig. 1. Release liner 6 may be in the shape of a circle with a half-moon shaped tab 6a extending out from its edge for easy removal. Release liner 6 has a diameter of 6.5 inches (16.5 cm), a thickness of 5 mils (0.013 cm), and a tab diameter of 1 inch (2.5 cm) in the exemplary embodiment shown.

Although the components described above in connection with Figs. 1-4C are circular, they may be rectangular (or any other shape). The reduced waste of a rectangular shape rather than a circular shape saves approximately 33 % in manufacturing costs. Thus, substrate 1, foil plate 2, retaining ring 4, and release liner 6 could have a rectangular shape with exemplary dimensions of approximately 6 inches (15.25 cm) by 3.75 inches (9.525 cm).

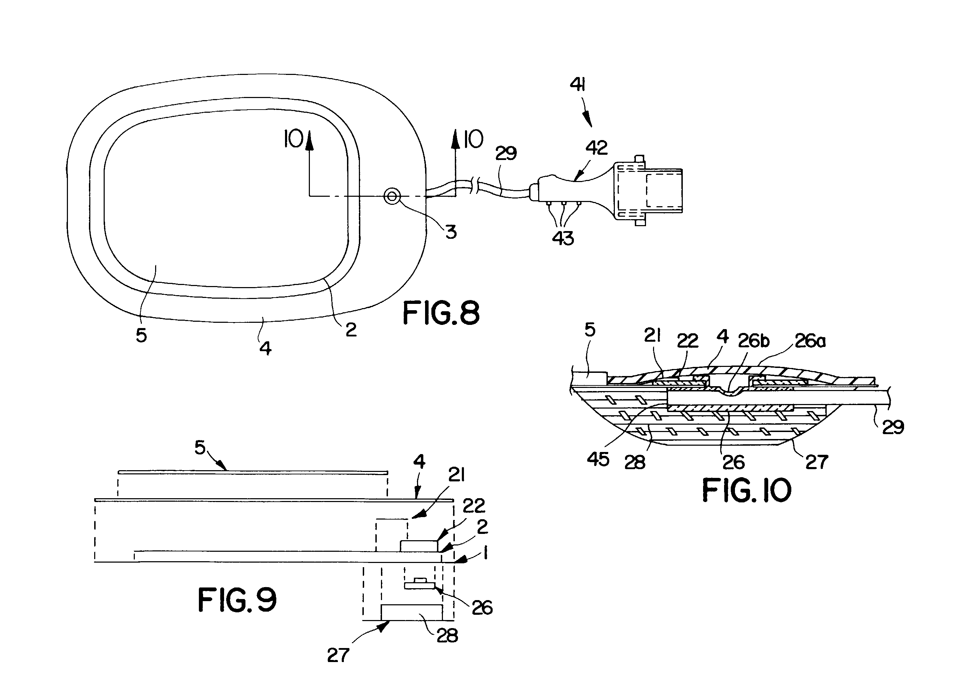

The electrode of the present invention may also have the connector structure used on the commercially available Hewlett Packard defibrillator electrode, as schematically illustrated in Fig. 3. In that structure, wires 30 and 31 (which may be attached to one another and split apart to extend to one of two electrodes 10 as illustrated) are pre-attached to electrodes 10, and a connector or lead assembly 33 is attached to the end of wires 30, 31 distal to electrodes 10 (described further below in connection with Figs. 9 and 10). Lead assembly 33 mates with another connector or plug (not shown) which has associated wires attached to a defibrillator machine (also not shown).

An alternative embodiment of the electrode of the present invention having a rectangular, or oblong, shape is shown in Fig. 5. Hydrogel 5 is contained within retaining ring 4 over foil plate 2 generally as described in connection with Fig. 1. Hole 3 is formed through the components of the electrode for attaching a connector, described hereinafter, to the electrode. Fig. 5A shows a top view of metal foil plate 2. As shown in Fig. 5A, foil plate 2 has an extended portion or tab 2a with hole 3 formed through it.

Fig. 6 is a side assembly view showing the various components forming the electrode shown in Fig. 5. Substrate 1 has foil plate 2 adhered to a first surface thereof by a conventional adhesive. Hole 3 formed in foil plate 2 is aligned with a corresponding hole formed in substrate 1. A post 23, used as the snap connector in this embodiment, is aligned and inserted into hole 3 in the attached combination substrate 1 and foil plate 2. Washer 22 is placed over hole 3 in foil plate 2 such that post 23 extends upward through washer 22. Washer 22 makes electrical contact between post 23 and foil plate 2. Washer 22 also secures post 23 to foil plate 2 after post 23 is crimped around washer 22 (as described below) in order to sandwich washer 22 and foil plate 2 together. A strip of double-sided tape 21 is then placed across metal foil 2 and washer 22, and is used to adhere retaining ring 4 to metal foil 2. Hydrogel 5 is then placed within retaining ring 4.

Fig. 7 shows a side sectional view taken along line 7-7 of the electrode depicted in Fig. 5. As shown in Fig. 7, the top 23a of post 23 is inserted through hole 3 in substrate 1 and metal foil 2. After washer 22 is placed over top 23a of post 23 extending through hole 3, top 23a of post 23 is crimped down over washer 22 as shown. Crimping or rolling top 23a of post 23 over washer 22 secures washer 22 to metal foil 2 and substrate 1. Crimping top 23a of post 23 over washer 22 thus allows secure attachment without the necessity of a separate part. Crimping top 23a of post 23 over washer 22 provides positive, reliable, low resistance contact between washer 22, post 23, and foil plate 2. The securement of retaining ring 4 to metal foil 2 using double-sided tape 21 is also illustrated.

Also shown in Fig. 7 are certain advantageous features of post 23. In particular, spherical ball 25 on the end of post 23 renders attachment of post 23 to a plug (not shown) easier than with known devices. Specifically, because the electrodes of the present invention are typically used in emergency situations in which rapid assembly of the device and rapid attachment to electrical connections is required, a user may simply place spherical ball 25 in the vicinity of an appropriate outlet in the attachment plug and apply force. The spherical design of ball 25 causes it to slide into exact alignment and into connection with the plug more readily than other designs requiring more precise alignment by the user. In addition, the plug, upon having post 23 inserted into it, will first expand as spherical ball 25 enters it, and then contract around spherical ball 25, thereby more securely locking post 23 within the plug.

Indentations 24 and post 23 are also adapted to hold post 23 in the connector plug more securely than previous devices. As with the locking mechanism described in connection with ball 25, a corresponding portion of the plug will expand as post 23 is inserted into it and then contract into indentations 24, thus locking post 23 in position.

Washer 22 shown in Fig. 7 must be of adequate size such that repeated attachment and detachment of the plug from post 23 does not tear foam retaining ring 4 or substrate 1. Washer 22 must also be wide enough to distribute sufficiently the load from such attachment and detachment around hole 3 in substrate 1 and metal foil 2 to prevent damage to those components.

Fig. 8 shows an alternative electrode according to the present invention. The main difference between the electrode shown in Fig. 8 and that of Fig. 5 is the design of the post used to make electrical connection between the electrode and an electrical source. Hydrogel 5 is contained within retaining ring 4 over metal foil plate 2 as described above, but the electrical connection is made using a lead wire assembly 41 attached to a cable 29 as shown in Fig. 8. Lead wire assembly 41 comprises an indentation 42 and ribs 43 that facilitate rapid, efficient, and secure attachment of cable 29 to the electrode. Specifically, a user's thumb fits in either indentation 42 or on ribs 43, and an opposing finger fits on the other of indentation 42 and ribs 43 to enable a swift and sure grip on lead wire assembly 41. This is useful for rapid attachment of cable 29 and is a significant advance in reducing the time necessary to assemble the parts of the electrode. Lead wire assembly 41 thus has a ergonomic design not heretofore achieved in known devices. The ergonomic design of the lead wire assembly gives it finger-grip capability making it easier to grip, which is important in emergency situations wherein the wire 29 must be easily inserted and twisted for attachment.

Fig. 9 is a side assembly view of the electrode shown in Fig. 8. Foil plate 2 is secured to substrate 1, and connector 26 is inserted through the aligned holes 3 in substrate 1 and foil plate 2. Washer 22 is then placed over the portion of connector 26 extending through hole 3. Double-sided tape 21 is placed over metal foil 2 and washer 22 to secure retaining ring 4 to metal foil plate 2. Hydrogel 5 is placed within retainer ring 4. An insulating foam 28 is placed around connector 26 so that an operator does not touch connector 26 with his or her hand. A tongue 27 is an adhesive label used to secure insulating foam 28 over connector 26 to substrate 1. Insulating foam 28 may be a polyether ultracel, such as that available from Par-Foam of Buffalo, New York. Tongue 27 functions not only to secure insulating foam 28 in position, but may be used as a label that identifies the product or other information relevant to the device.

Fig. 10 is a side sectional view taken along line 10--10 of the electrode shown in Fig. 8. Figs. 10-12 show connector 26 used for attaching wire 29 to the electrode. The top 26a of electrode 26 extends through the hole in substrate 1 and metal foil 2. Top 26a is then crimped around washer 22 as described in connection with the previous embodiment.

Connector 26 is formed in the shape of a spherical button of conductive material. Connector 26 has a hole 45, which preferrably has a stepped diameter for securing wire 29 therein, extending diametrically therethrough, into which wire 29 may be inserted. Once wire 29 is inserted, connector 26 is crimped to form indentation 26b which secures wire 29 within connector 26. Connector 26 provides a secure crimp of wire 29 and holds wire 29 with a one-piece construction, providing advantages over two-piece connector constructions heretofore known and available.

Also in Fig. 10, insulating foam 28 is shown to cover connector 26. Tongue 27 securely attaches insulated foam 28 to the bottom of substrate 1.

Fig. 13 illustrates a side assembly view of an alternative electrode according to the present invention. Substrate 1, foil plate 2, retaining ring 4, hydrogel 5, and washer 22 are assembled generally as described above in connection with other embodiments. In this embodiment, however, a cup 50 is used as the connector for the electrode. Cup 50 is inserted through hole 3 in substrate 1 and foil plate 2 and crimped around washer 22 as. described above in connection with other embodiments. An insulating spacer 51 is inserted over cup 50 to provide a buffer that fills the space between a plug (not shown) that is attached to cup 50 and substrate 1. Insulating spacer 51 prevents an operator from getting his or her finger caught between the plug and substrate 1, and prevents operator contact with cup 50. Insulating spacer 51 is typically made of foam.

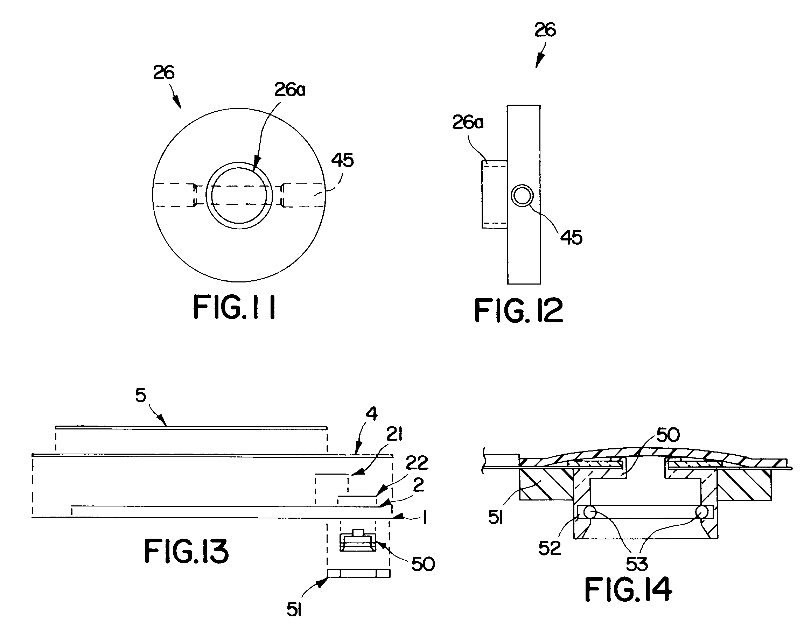

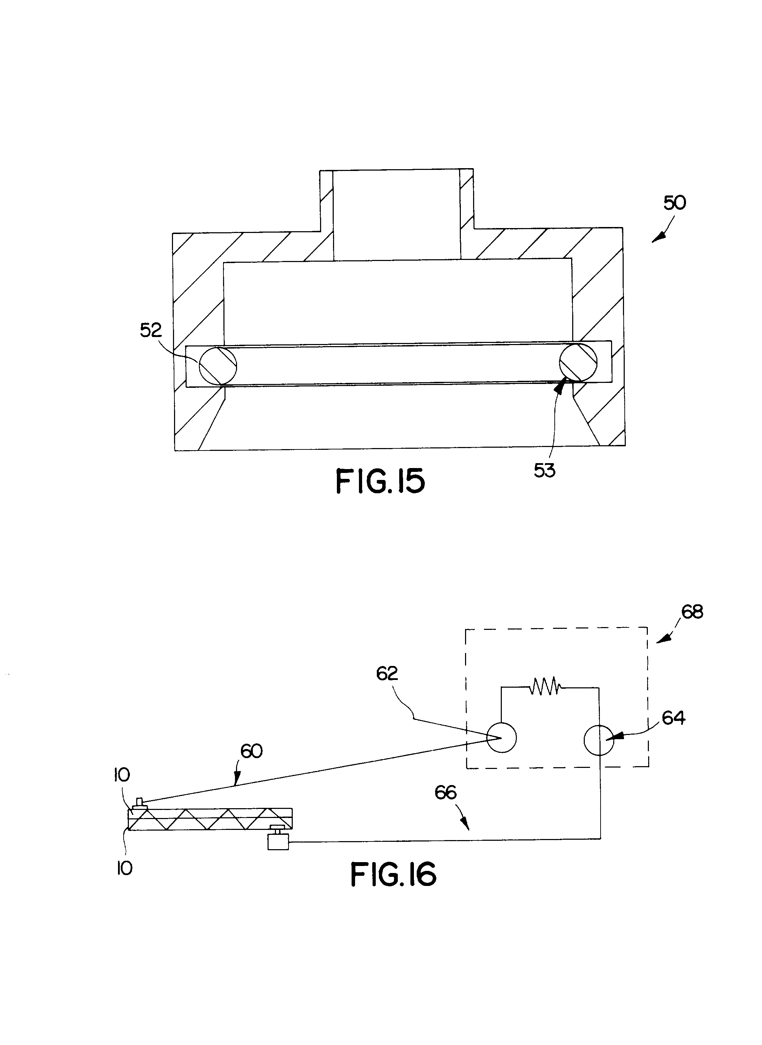

As shown in Figs. 14 and 15, cup 50 has a groove 52 formed therein, which contains a snap ring 53. Snap ring 53 may be made of stainless steel and have a portion thereof removed to enable contraction and expansion. Snap ring 53 is contained within groove 52 leaving a gap between snap ring 53 and cup 50 when no plug is attached to cup 50. Upon attachment of a plug, snap ring 53 expands and contacts the walls of cup 50, but exerts an inward force securing the plug within cup 50. This provides a stable and secure attachment of cup 50 to the plug.

Exemplary electrodes of the present invention such as those described in connection with Fig. 1 were tested for electrical properties using the test circuit schematically illustrated in Fig. 16. Electrodes 10 were connected gel-to-gel. A DynaTech Tester 68 with an internal 50 ohm load was connected to electrodes 10. Banana plugs 62 and 64 were connected by jumper wires 60 and 66, respectively, to the center post of electrodes 10.

Three sample pairs of electrodes were connected gel-to-gel in series as described above with a 50 ohm load. The impedance for each sample pair was measured at 200 joules and 360 joules. The results are presented in the following table. These results illustrate satisfactory performance of the electrodes.

| **Sample** | **Impedance at 200 Joules** | **Impedance at 360 Joules** |
|---|---|---|
| 1 | .46 OHMS | .51 OHMS |
| 2 | .20 OHMS | .25 OHMS |
| 2 | .75 OHMS | .150 OHMS |

Although illustrated and described herein with reference to certain specific embodiments, the present invention is nevertheless not intended to be limited to the details shown. Rather, various modifications may be made into details within the scope and range of equivalent claims and without departing from the spirit of the invention.

## Claims

1. A defibrillator electrode comprising
(a) a substrate having a periphery,
(b) a foil plate adhered to said substrate,
(c) a retaining ring defining a volume adhered around said periphery of said substrate,
(d) a conductive hydrogel adhesive containing an electrolyte disposed over said foil plate within said volume of said retaining ring, and
(e) a release liner disposed over said conductive hydrogel adhesive.

2. A defibrillator electrode as claimed in claim 1 wherein said electrolyte is potassium bromide.

3. A defibrillator electrode as claimed in claim 1 further comprising means for making an electrical connection with said electrode.

4. A defibrillator electrode as claimed in claim 3 wherein said means for making electrical connection is a post having a substantially cylindrical portion with an end, an indentation on said spherical portion, and a spherical ball on said end, wherein said indentation and said spherical ball are adapted to mate with corresponding portions of a plug to which said post is connected.

5. A defibrillator electrode as claimed in claim 3 wherein said means for making electrical connection is a post having a substantially disk-shaped body portion and a top portion disposed on said body portion, said body portion having a hole extending diametrically therethrough to receive a wire for establishing electrical connection with said electrode.

6. A defibrillator electrode as claimed in claim 3 wherein said means for making electrical connection is a post having a substantially cup-shaped body portion having a wall, a groove in said wall, and a snap ring disposed in said groove.

7. A defibrillator electrode as claimed in claim 3 wherein said means for making electrical connection comprises a post having a portion adapted to extend through a hole in said electrode and to be crimped thereon.

8. A defibrillator electrode as claimed in claim 3 further comprising means for making electrical contact between said foil plate and said means for making electrical connection.

9. A defibrillator electrode as claimed in claim 8 wherein said means for making electrical contact comprises a washer sized to distribute load over a portion of said foil plate.

10. In a lead wire assembly for making an electrical connection between a wore and an electrical source wherein the assembly has a first portion adapted to be connected to the wire and a second portion integral to the first portion and adapted to be connected to the electrical source, the improvement comprising an indent formed on said first portion and a plurality of ribs formed on said first portion diametrically opposite said indent, wherein a user's fingers are adapted to be placed on said indent and said ribs for efficient manipulation of said lead wire assembly.
